# EUROPEAN PATENT APPLICATION

(11) **EP 2 105 145 A1**
(43) Date of publication of application: **30.09.2009**
(21) Application number: 08005800.1
(22) Date of filing: 27.03.2008
(51) Int. Cl.: A61K 47/48

(54) **Method for muscle-specific delivery lipid-conjugated oligonucleotides**

(71) Applicant: ETH Zurich, 8092 Zurich (CH)
(72) Inventor: Stoffel, Markus, 8044 Zürich (CH); Wolfrum, Christian, 8046 Zürich (CH)

(57) **Abstract**

The invention describes a method for muscle-specific delivery of oligonucleotides to induce gene silencing by RNA interference or silencing of microRNAs in vivo. A liposome comprising triacylglycerol, phospholipids, glycerol and lipid-binding proteins aggregated via a linker molecule with a single- or double-stranded oligonucleotide that is stabilized by one or more oligonucleotide modifications is isolated from Intralipid and serves as tissue-specific carrier.

SEQUENCE LISTING

<110>  ETH Zurich

<120>  Intralipid

<130>  T-07-103

<160>  5

<170>  PatentIn version 3.5

<210>  1
<211>  21
<212>  RNA
<213>  Artificial

<220>
<223>  siRNA-apoB1, sense strand

<400>  1
gucaucacac ugaauaccaa u                                                21

<210>  2
<211>  23
<212>  RNA
<213>  Artificial

<220>
<223>  siRNA-apoB1, antisense strand

<400>  2
auugguauuc agugugauga cac                                              23

<210>  3
<211>  22
<212>  RNA
<213>  Artificial

<220>
<223>  antagomir-16

<400>  3
cgccaauauu uacgugcugc ua                                               22

<210>  4
<211>  21
<212>  RNA
<213>  Artificial

<220>
<223>  antagomir-133a

<400>  4
auuugguucc auuuuaccag c                                                21

<210>  5
<211>  22
<212>  RNA
<213>  Artificial

<220>
<223>  antagomir-206

<400> 5
ccacacacuu ccuuacauuc ca 22

## Description

### FIELD OF THE INVENTION

The present invention relates to a carrier for oligonucleotides and to tissue-specific delivery of the oligonucleotides to heart, lung and muscle cells.

### BACKGROUND OF THE INVENTION

Synthetic oligonucleotides have emerged as an important tool for post-transcriptional gene silencing in mammalian cells and live animals due to their unique properties such as potency, specificity and lack of interferon response. Three different types of oligonucleotides are distinguished based on the different mechanisms of gene silencing: dsRNA mediating RNA interference, ssRNA targeting non-coding and coding RNAs and ss oligonucleotides targeting proteins.

Double-stranded RNA (dsRNA) mediates RNA interference (RNAi). RNAi is an evolutionarily conserved and powerful biological process for specific silencing of gene expression. Small interfering RNA strands (siRNA) are key to the RNAi process, and have complementary nucleotide sequences to the targeted RNA strand. Specific RNAi pathway proteins are guided by the siRNA to the targeted messenger RNA (mRNA), where they "cleave" the target, breaking it down into smaller portions that can no longer be translated into protein. A type of RNA transcribed from the genome itself, microRNA (miRNA), works in the same way (Y. Dorsett & T. Tuschl 2004, Nature Reviews Drug Discovery 3, 318-329). The RNAi pathway is initiated by the enzyme *dicer*, which cleaves long dsRNA molecules into short fragments of 20-25 base pairs. One of the two strands of each fragment, known as the guide strand, is then incorporated into the RNA-induced silencing complex (RISC) and pairs with perfect complementary sequences. The best studied outcome of this recognition event is post-transcriptional gene silencing. This occurs when the guide strand specifically pairs with an mRNA molecule and induces the degradation by *argonaute*, the catalytic component of the RISC complex.

The second type of oligonucleotides is single-stranded RNA (ssRNA) targeting non-coding (e.g. microRNAs) and coding RNAs (mRNAs). microRNAs (miRNA) are single-stranded RNA molecules of about 21-23 nucleotides in length, which regulate gene expression. miRNAs are encoded by genes that are transcribed from DNA but not translated into protein (non-coding RNA); instead, they are processed from primary transcripts known as *pri-miRNA* to short stem-loop structures called *pre-miRNA* and finally to functional miRNA. Mature miRNA molecules are partially complementary to one or more messenger RNA (mRNA) molecules, and their main function is to down regulate gene expression (D.P. Bartel 2004, Cell 116, 281-297).

The third type is single-stranded oligonucleotides (ss oligonucleotides) targeting proteins (e.g. aptamers). Aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms. Aptamers offer the utility for biotechnological and therapeutic applications as they offer molecular recognition properties that rival that of the commonly used bio-molecule, antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications (D.H.J. Bunka & P.G. Stockley 2006, Nature Reviews Microbiology 4, 588-596).

Specific oligonucleotides of any of the three types can be designed for any endogenous gene, RNA or protein. In contrast to chemical compounds, it is possible to silence any gene or protein expression. Synthetic oligonucleotides are therefore predicted to be a very effective class of medicaments for the future. First successful medicaments are already on the market.

To date, delivery remains the largest obstacle for *in vivo* applications of synthetic oligonucleotides, including their use as therapeutics following systemic administration. Delivery of oligonucleotides across plasma membranes *in vivo* has been achieved using vector-based delivery systems, high-pressure intravenous injections of oligonucleotides and various chemically-modified oligonucleotides, including cholesterol-conjugated, lipid encapsulated and antibody-mediated oligonucleotides.

As an example, covalent conjugation of highly hydrophobic cholesterol to RNAs mediates cellular import of double-stranded RNAs and single-stranded antisense RNAs and elicits RNA interference and microRNA silencing, respectively. Cholesterol-conjugated RNAs bind to HDL and LDL lipoprotein particles which mediate cellular uptake upon binding to their respective receptors. Both, high-density lipoproteins (HDL) and low density lipoproteins (LDL) play a critical role in cholesterol transport. HDL directs siRNA delivery into liver, gut, kidney and steroidogenic organs, whereas LDL targets siRNA primarily to liver (Wolfrum et al. Nature Biotechnology Vol. 25 (2007)).

However, up to day, no method for muscle-specific delivery of lipid-conjugated RNAs exists.

Liposomes are, due to their unique properties, another widely used tool for transporting drugs (V. P. Torchilin, Nature Rev. Drug Discov. 2005, 4(2):145-160). A liposome is a spherical, self-closed structure formed by one or several concentric lipid bilayers with an aqueous phase inside and between lipid bilayers. Liposomes can vary in size, shape and composition. Traditionally, liposomes refer to a membrane composed of a phospholipid and cholesterol bilayer. Liposomes can be composed of naturally-derived phospholipids with mixed lipid chains like egg phosphatidylethanolamine, or of pure surfactant components like dioleoylphosphatidylethanolamine. A liposome encapsulates a region on aqueous solution inside a hydrophobic membrane; dissolved hydrophilic solutes can not readily pass through the lipids. Hydrophobic chemicals can be dissolved into the membrane, and in this way, liposomes can carry both hydrophobic molecules and hydrophilic molecules. To deliver the molecules to the site of action, the lipid bilayer can fuse with other bilayers such as the cell membrane, thus delivering the liposome contents.

Immunoliposomes have been designed for accumulation in desired tissues and organs. The use of targeted liposomes with surface-attached ligands capable of recognizing and binding to cells of interest has been suggested. Folate and transferrin mediates liposome targeting to tumor cells. Targeting tumors with folate- or transferrin-modified liposomes represents a promising approach, since folate and transferrin receptors are frequently over expressed in tumor cells.

Another source for liposomes is Intralipid. Intralipid® is a brand name for the first safe fat emulsion for human use, approved in 1962 and invented by Professor Arvid Wretlind, Sweden. It is given intravenously to patients who are unable to get enough fat in their diet. It is therefore completely non-toxic and well tolerated in human.

Intralipid® 20% (a 20% intravenous fat emulsion) is a sterile, non-pyrogenic fat emulsion prepared for intravenous administration as a source of calories and essential fatty acids. It is made up of 20% soybean oil, 1.2% egg yolk phospholipids, 2.25% glycerol, and water for injection. In addition, sodium hydroxide has been added to adjust the pH so that the final product pH is about 8, ranging from 6 to 8.9. Intralipid® 20% has an osmolality of approximately 350 mosmol/kg water (which represents 260 mosmol/liter of emulsion) and contains emulsified fat particles of approximately 0.5 micron size.

The soybean oil is a refined natural product consisting of a mixture of neutral triglycerides of predominantly unsaturated fatty acids.

The major component fatty acids are linoleic acid, C₁₈H₃₂O₂ (44-62%); oleic acid, C₁₈H₃₄O₂ (19-30%); palmitic acid, C₁₆H₃₂O₂ (7-14%); linolenic acid, C₁₈H₃₀O₂ (4-11%) and stearic acid, C₁₈H₃₆O₂ (1.4-5.5%). Soybean oil additionally contains traces of lauric acid, myristic acid, arachidic acid and palmitioleic acid.

Purified egg phosphatides are a mixture of naturally occurring phospholipids which are isolated from the egg yolk. These phospholipids contain saturated and unsaturated fatty acids and either the choline or ethanolamine ester of phosphoric acid.

Glycerol is chemically designated C₃H₈O₃ and is a clear colorless, hygroscopic syrupy liquid.

The present invention relates to a method for delivering oligonucleotides into muscle *in vivo* and for the treatment of muscle diseases.

### DESCRIPTION OF THE INVENTION

The technical problem to be solved was to provide a non-toxic carrier for oligonucleotides that is targeting the oligonucleotides specifically to heart, lung and muscle tissues.
The problem is solved by the liposome according to claim 1. Further preferred embodiments are subject of claims 2 to 13.
Many liposomes are toxic, in part because of stimulating inflammatory cytokine release, and their application is therefore limited for medical use as drug delivery system. Several sources of liposomes were tested. The problem is solved by a particular liposome from Intralipid® emulsion. Intralipid is completely untoxic and approved for medical application.

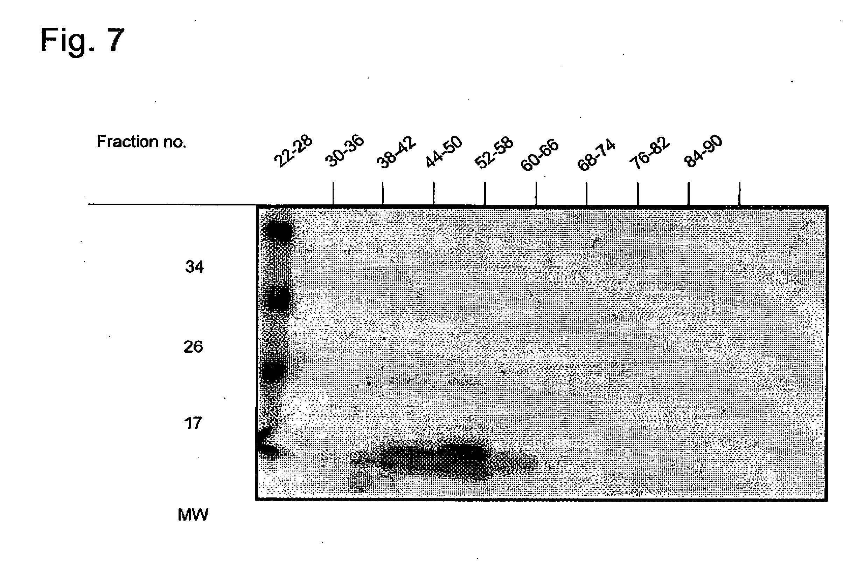

Analysis of Intralipid by FPLC revealed that this emulsion contains various stable lipid particles of distinct size. One specific liposome, obtainable by fractionating Intralipid, has an affinity to heart, lung and muscle tissues. This specific liposome is called Intralipid #1. The lipid composition of Intralipid #1 is similar as the complete Intralipid mixture but comprises one or several specific proteins of MW between 10 and 20 kD (Figure 7). The Intralipid used for the invention was manufactured by Fresenius Kabi, Uppsala Sweden (100ml Intralipid 20% comprising 20g Soybean oil, 1.2g Phospholipid and 2.25g Glycerol (USP)).

According to claim 1, the liposome of the invention comprises triacylglycerol, phospholipids, glycerol and one or several lipid-binding proteins aggregated via a lipophilic linker molecule with a single- or double-stranded oligonucleotide, wherein said liposome has an affinity to heart, lung and/or muscle tissue. Surprisingly, it has been found that due to said one or several lipid-binding proteins in combination with the above mentioned lipids, the affinity to heart, lung and/or muscle tissue is very specific. These liposomes serve as carrier for oligonucleotides. Due to their affinity to heart, lung and muscle cells, they specifically transport the oligonucleotides to these tissues. Therefore, the liposome aggregates according to the present invention may be used for many severe heart, lung and muscle diseases, for example myocarditis, ischemic heart disease, myopathies, cardiomyopathies, metabolic diseases, rhabdomyosarcomas.

The oligonucleotide is preferably stabilized by one or more oligonugleotide modifications to avoid degradation of the oligonucleotides. The nuclease resistance of modified oligonucleotides is increased compared to a sequence containing only unmodified ribonucleotide moieties, unmodified desoxyribonucleotide moieties or both. Such modified moiteties are well known in the art (Kurreck, European Journal of Biochemistry 270 (2003), 1628-1644). In one example, the oligonucleotide molecule comprises at least one modified moiety. Possible modifications are for example Phosporothioate DNA units, 2'-O-methyl RNA units, 2'-O-methoxy-ethyl RNA units, Peptide nucleic acid units, N3'-P5' Phosphoroamidate DNA units, 2' fluoro-ribo nucleic acid units, Locked nucleic acid units, Morpholino phosphoroamidate nucleic acid units, Cyclohexane nucleic acid units, Tricyclonucleic acid units, 2'-O-alkylated nucleotide modifications, 2'-Deozy-2'-fluoro modifications, 2,4-difluorotoluyl modifications, 4'-Thio ribose modifications, and Boranophospate modifications. Especially preferred are 2'-O-methyl units, Phosporothioate units, Locked nucleic acid units and 2' fluoro-ribo nucleic acid units. Most preferred are 2'-O-methyl units.

A preferred embodiment of the invention is a liposome comprising 15-25% triacylglycerol, 0.5-2% phospholipids and 1-3 % glycerol, and one or several lipid-binding proteins.

A more preferred embodiment of the invention is a liposome comprising about 20% triacylglycerol, about 1.2% phospholipids and about 2.25% glycerol, which corresponds to the total composition of Intarlipid, and one or several lipid-binding proteins.

An even more preferred embodiment of the invention is Intralipid #1.

In a preferred embodiment, the liposome aggregate has a particle size of 20-50 nm, more preferably of about 35 nm.

The oligonucleotides are aggregated with the liposomes via a linker molecule at the outer surface of the liposome. Any lipophilic linker molecule that is able to bind oligonucleotides to lipids can be chosen. Preferred linkers are Pyrrolidine and Hydroxyprolinol. Most preferred is Cholesterol.

Any single- or double-stranded oligonucleotide that is able to specifically interact with gene expression, RNA or protein activity may be linked to the liposomes. Preferred oligonucleotides have an interacting gene, RNA or protein in heart, lung and muscle cells. Oligonucleotides are normally 10-40 nucleotides in length, preferably 15-30, most preferably 20-25 nucleotides.

Preferred oligonucleotides are oligonucleotides mediating RNA interference, oligonucleotides targeting RNAs and oligonucleotides targeting proteins. Most preferred oligonucleotides are single- and double-stranded RNAs.

The invention further relates to selecting the liposomes from the Intralipid emulsion. Any method fractionating the Intralipid emulsion and separating the different lipid particles can be chosen. Particles of 20-50nm, preferably of about 35nm in size are selected. Possible fractionating methods are liquid chromatography or ultracentrifugation.

The process for making the liposomes comprises the steps of:
a) mixing a lipid emulsion with one or several single- or double-stranded lipophile (e.g. cholesterol) conjugated oligonucleotides that may be chemically modified;
b) fractionating this mixture;
c) selecting the fraction with particles of 20-50nm, preferably of about 35nm in size.

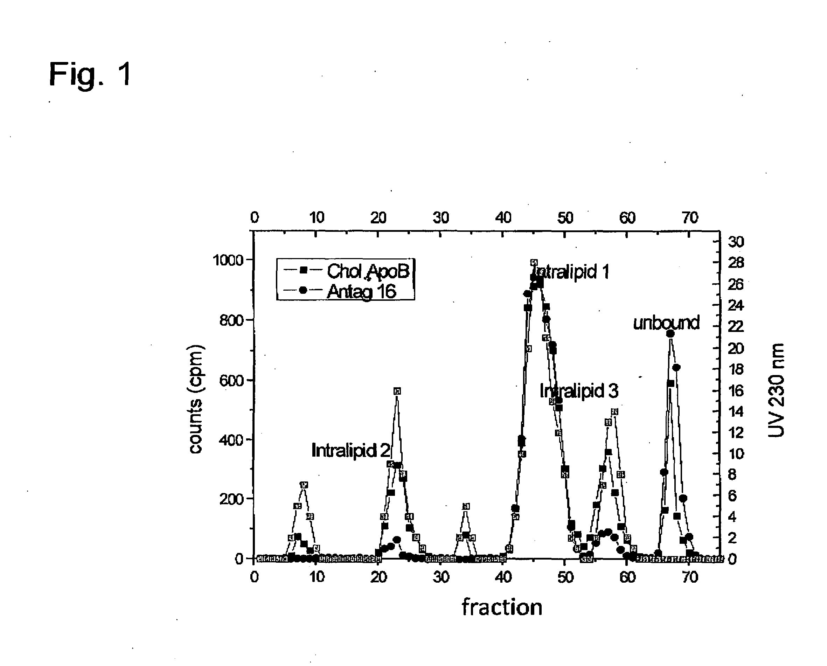

As an example, Cholesterol-conjugated RNAs (Chol-apoB1 and Antagomir-16) bind to Intralipid peak #1 that elutes between fraction 40 to 50 (Figure 1), containing particles between 20 and 50nm in size.

Alternatively, the liposomes can be made by first isolating the lipid particles comprising triacylglycerol, phospholipids, glycerol and one or several lipid-binding proteins and corresponding to Intralipid #1 particles and then mixing the isolated particles with >2-fold molar excess of single- or double-stranded lipophile (e.g. cholesterol) conjugated oligonucleotide. The steps of fractionating and selecting the particles are deleted by this alternative process for making the liposomes.

The liposome aggregates according to the present invention are used as medicament. Preferably, they are used to treat heart, lung or muscle diseases. Such medicaments are primarily used to treat disorders caused by overexpression of particular genes, RNAs or proteins.

The invention further relates to a lyophilisat containing the liposome aggregate.

In another aspect, the invention relates to a method for selectively targeting a single-or double-stranded oligonucleotide to mammalian heart, lung and/or muscle tissues comprising contacting a mammal with said oligonucleotide, wherein said oligonucleotide has been formulated via a lipid emulsion fractionation process according to the description above.

Yet another aspect relates to a method for reducing expression of a gene in mammalian tissue *in vivo* comprising contacting said tissue with the liposome according to this invention.

### FIGURES

**Figure 1****:** Analysis of Intralipid® by FPLC revealed that this emulsion contains various stable lipid particles of distinct size. Cholesterol-conjugated RNAs (Chol-apoB1-siRNA and antagomir-16) bind to Intralipid peak#1 that elutes between fraction 40 to 50. Intralipid 1 corresponds to the liposome aggregate of the invention. RNAs were labelled with ³²P and lipid fractions were determined by UV absorbance at 230 nm.

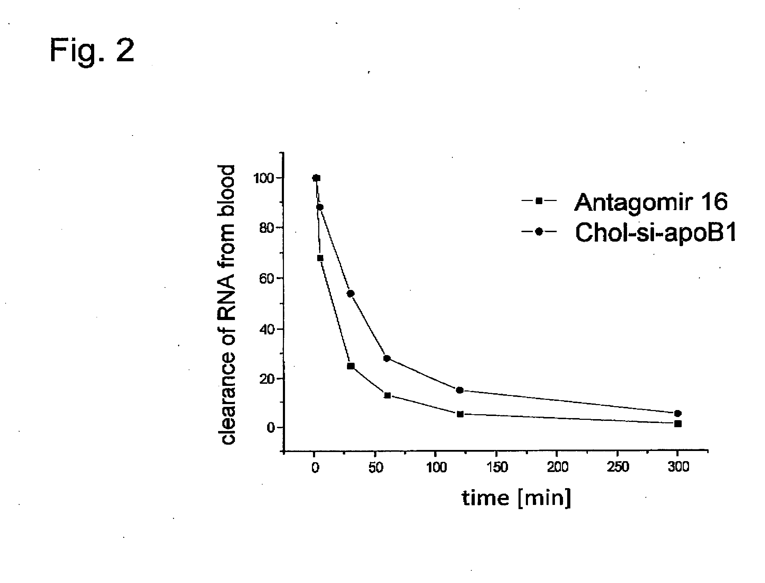

**Figure 2****:** Plasma clearance of Intralipid/antagomir-16 and Intralipid/chol-apoB1-siRNA following tail injections. The blood t1/2 for antagomir-16 is ≈20 min, t1/2 of chol-apoB1-siRNA ≈40. The experiment shows rapid removal of Intralipid/RNA complexes from the blood after systemic injection.

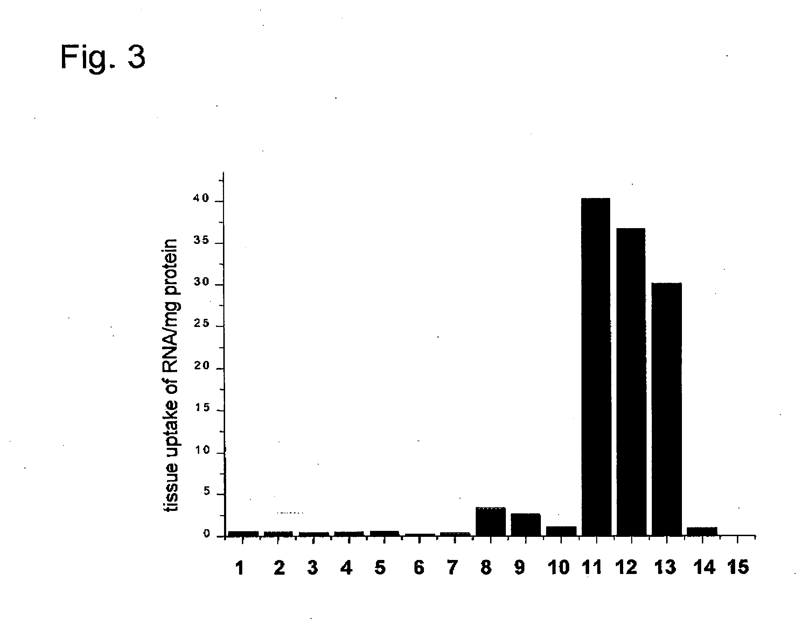

**Figure 3****:** Tissue uptake of ³²P-labeled chol-apoB1-siRNA mediated by "Intralipid 1° 4h after systemic injection. Uptake is normalized/mg protein. The experiment shows selective (enriched) uptake of Intralipid/chol-siRNA in muscle, heart and lung.
- Lane 1: liver
- Lane 2: stomach
- Lane 3: duodenum
- Lane 4: jejunum
- Lane 5: ilium
- Lane 6: colon
- Lane 7: testis
- Lane 8: kidney
- Lane 9: adrenal
- Lane 10: spleen
- Lane 11: heart
- Lane 12: muscle
- Lane 13: lung
- Lane 14: fat
- Lane 15: brain

**Figure 4****:** Silencing activity of chol-apoB1-siRNA following chol-apoB1-siRNA/Intralipid 1 injection.
ApoB mRNA expression levels in the heart of mice injected with a single injection of chol-apoB1-siRNA at 10 mg/kg bodyweight were measured by real time PCR 24h after injection. Wildtype denoted PBS control, Chol-apoB unconjugated indicates injection of siRNA in the absence of Intralipid 1. Chol-apoB Intralipid 1 denotes siRNAs that were complexed with Intralipid 1 prior to injection.
- Lane 1:: wildtype
- Lane 2:: Chol ApoB unconjugated
- Lane 3: Chol ApoB Intralipid 1

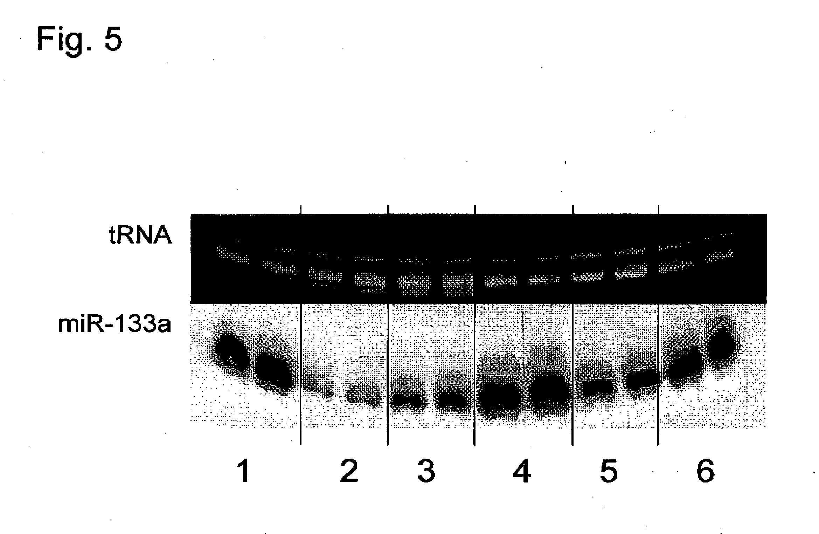

**Figure 5****:** Silencing of microRNA-133a (miR-133a) following antagomir-133a/Intralipid#1 injection: miR-133a levels in heart and quadriceps muscle in response to antagomir-133a/Intralipid injection (50mg/kg bodyweight). miR-133a levels in heart and quadriceps muscle of mice injected with a single injection of antagomir-133a at 50 mg/kg bodyweight (duplicates for each treatment). Expression levels were measured by Northern blotting 24h after injection. tRNA levels are shown as a loading control. Antagomir (ant.133a) indicates injection of antagomir in the absence of Intralipid 1. Intralipid ant.133a denotes antagomir-133a that was complexed with Intralipid 1 prior to injection.
- Lanes 1 and 4:: PBS
- Lanes 2 and 5:: Intralipid ant.133a
- Lanes 3 and 6:: ant.133a
- Lanes 1-3: represents heart tissue
- Lanes 4-6: represents M.quadriceps (50 mg/kg bodyweight)

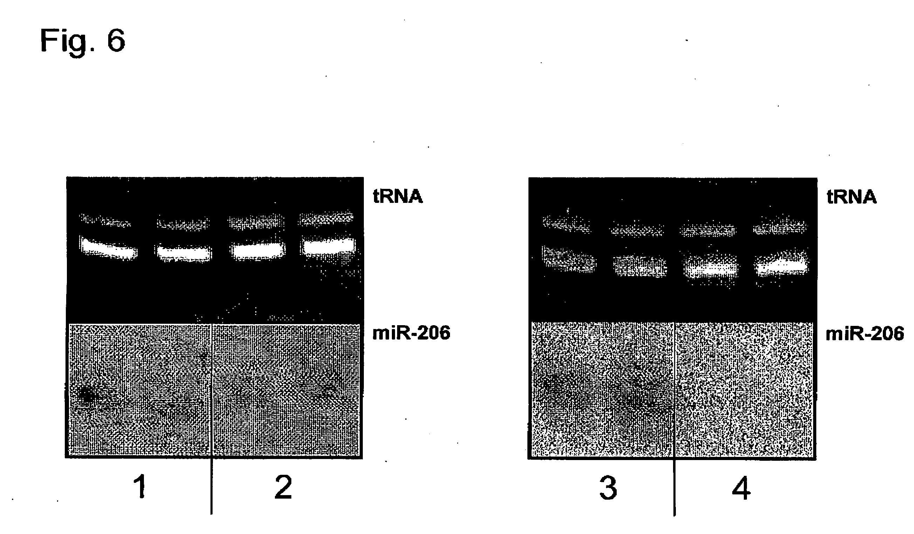

- **Figure 6:**: Targeting Muscle-specific miR-206 with a lipid-antagomir complex
- Lane 1:: control (3x 80mg/kg bodyweight i.v.)
- Lane 2:: antagomir-206 (3x 80mg/kg bodyweight i.v.)
- Lane 3:: control (3x 50mg/kg bodyweight i.v.)
- Lane 4:: Intralipid/antagomir-206 (3x 50mg/kg bodyweight i.v.)

**Figure 7****:** Low molecular weight proteins are associated with Intralipid peak#1. Two protein bands at molecular weight (MW) between 10 and 20 kD can be detected in fractions 38-50 (corresponding to the fractions containing Intralipid#1).

### EXAMPLES

### Example 1

Intralipid (Sigma 20%) concentration is 200 mg/ml (approx. 570 µM). Intralipid is diluted to 133 µM and incubated 1:2 with Chol-conjugated RNA (Chol-RNA) (4 mg/ml, 266 µM, spiked with a small amount of ³²P labeled Chol-RNA for quantification) for 30 min at room temperature. Unbound Chol-RNA is separated by gel filtration using two Suprose 6 column (volume of run: 0.5ml) (Pharmacia HR10/30 code #17-0537-01) with FPLC running buffer (0.15 M NaCl, 0.01 M Na₂HPO₄, 0.1 mM EDTA, pH 7.5). The Intralipid/chol-RNA fraction is collected (particles between 20 to 50 nm) and Chol-RNA content quantified by scintillation counting.

### Example 2

Chol-apoB1-siRNA, a chemically-modified, lipophile-conjugated siRNA-apoB1, was constituted from the corresponding sense strand: 5'-GUCAUCACACUGAAUACCAAUₛHyp-L -3' and antisense strand: 5'-AUUGGUAUUCAGUGUGAUGAcₛaₛC -3' was obtained as described in Soutschek et al. 2004, Nature 432, 173-8. The cholesterol-conjugated sense strand was synthesized from a hydroxyprolinol-lipophile solid support. Cholesterol was tethered to *trans*-4-hydroxyprolinol via a 6-aminohexanoate linkage to obtain a hydroxyprolinol-lipophile moiety (Hyp) that was subsequently attached to a functionalized control pore glass to obtain the solid support. The letter 'L' represents the lipophile, lower case letters represent 2'-O-methyl sugar modification and subscript 's' stands for phosphorothioate linkages. The antagomir-16 used consisted of 21-23 nt length with modifications as specified: 5'-cₛgₛccaauauuuacgugcugₛcₛuₛaₛ-Chol-3'. The antagomir-133a used consisted of 21-23 nt length with modifications as specified: 5'-aₛuₛuugguuccauuuuaccₛaₛgₛcₛ-Chol-3'. The lower case letters represent 2'-O-methyl modified nucleotides; subscript 's' represent phosphorothioate linkage.

### Example 3

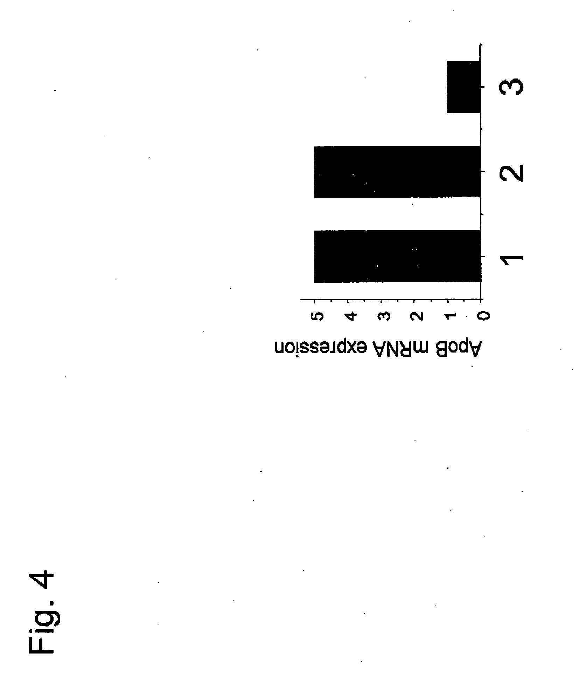

ApoB mRNA expression levels in the heart of mice injected with a single injection of chol-apoB1-siRNA at 10 mg/kg bodyweight were measured by real time PCR 24h after injection. A five-fold decrease of ApoB mRNA compared to control experiments shows the effectiveness of post transcriptional gene silencing (Figure 4).

### Example 4

Mice were injected with a single injection of antagomir-133a/Intralipid#1at 50 mg/kg bodyweight. Expression levels of microRNA-133a (miR-133a) were measured by Northern blotting 24h after injection.
The experiment shows *in vivo* silencing of miR-133a following antagomir-133a/Intralipid#1 injection in heart and quadriceps (Figure 5).

### Example 5

Mice were injected in duplicates with either PBS (control), antagomir-206, or antagomir-206 that was complexed with Intralipid#1 at a dose of 3x80 mg/kg body weight (bw) or 3x50 mg/kg, respectively. Mice were sacrificed on day 4 (24 h after the last injection), total RNA was isolated from the quadriceps muscle, and miR-206 levels were analyzed by Northern blotting. tRNA is shown as a loading control.
The antagomir-206 used consisted of 22 nt length with modifications as specified: 5'-cₛcₛacacacuuccuuacauuₛcₛcₛaₛ-Chol-3'. The lower case letters represent 2'-4-methyl modified nucleotides; subscript 's' represent phosphorothioate linkage.
The experiment shows complete *in vivo* silencing of miR-206 following antagomir-206/Intralipid#1 injection in quadriceps (Figure 6).

### Example 6

Intralipid was fractionated by HPLC, proteins were extracted from indicated fractions and separated on a 12% polyacrylamide gel. Proteins were visualized by silver staining. Two protein bands at molecular weight (MW) between 10 and 20 kD can be detected in fractions 38-50 (corresponding to the fractions containing Intralipid#1 (Figure 7).

## Claims

1. A liposome comprising triacylglycerol, phospholipids, glycerol and one or several lipid-binding proteins aggregated via a lipophilic linker molecule with a single- or double-stranded oligonucleotide, wherein said liposome has an affinity to heart, lung and/or muscle tissue.

2. The liposome according to claim 1, wherein the oligonucleotide is stabilized by one or more oligonucleotide modifications.

3. The liposome according to any of claims 1 or 2, comprising about 20% triacylglycerol, about 1.2% phospholipids and about 2.25% glycerol and one or several lipid-binding proteins.

4. The liposome according to any of the preceding claims having a particle size of 20-50 nm, preferably of about 35 nm.

5. The liposome according to any of the preceding claims, wherein the oligonucleotide is an oligonucleotide mediating RNA interference, an oligonucleotide targeting RNAs or and oligonucleotide targeting proteins.

6. The liposome according to any of the preceding claims, wherein the oligonucleotide is an RNA.

7. The liposome according to any of the preceding claims obtainable by fractionating an emulsion of lipid particles and selecting for particles of 20-50 nm, preferably of about 35 nm in size.

8. A process for making the liposome aggregate according to any of claims 1 to 7 comprising:
a) mixing a lipid emulsion with one or several single- or double-stranded oligonucleotides;
b) fractionating the mixture of step a);
c) selecting the fraction with particles of 20-50 nm, preferably of about 35 nm in size.

9. A process for making the liposome aggregate according to any of claims 1 to 7, wherein the lipid particle comprising triacylglycerol, phospholipids, glycerol and one or several lipid-binding proteins is first isolated from a lipid emulsion and then mixed with >2-fold molar excess of one or several lipophile conjugated single- or double-stranded oligonucleotide.

10. The liposome according to any of the claims 1 to 7 for the use as a medicament.

11. The liposome according to any of the claims 1 to 7 for the treatment of heart, lung and/or muscle diseases.

12. Use of the liposome according to any of the claims 1 to 7 for the manufacture of a medication for the treatment of heart, lung and/or muscle diseases.

13. A lyophilisat comprising the liposome according to any of the claims 1 to 7.
